# EUROPEAN PATENT APPLICATION

(11) **EP 4 800 438 A1**
(43) Date of publication of application: **02.09.2026**
(21) Application number: 25160475.7
(22) Date of filing: 27.02.2025
(51) Int. Cl.: G01T 1/24, G01N 23/2251, G01T 1/29, G01T 1/36

(54) **SPECTRALLY RESOLVING SEMICONDUCTOR X-RAY DETECTOR WITH LIGHT EMITTING LAYER AND METHOD THEREFOR**

(71) Applicant: Carl Zeiss X-Ray Microscopy, Inc., Dublin, California 94568 (US)
(72) Inventor: Xu, Xiaochao, Dublin, 94568 (US); Andrew, Matthew, Dublin, 94568 (US)
(74) Representative: Gulde & Partner

(57) **Abstract**

A detection system comprises a semiconductor layer for converting x-ray photons or particles into charge carriers and a light emitting layer for generating light from the charge carriers. A computer system obtains images generated by the camera and tracks the response of the detector to x-ray photons and/or charged particles over time in order to spectrally resolve the energy of the x-ray photons or particles.

## Description

### BACKGROUND OF THE INVENTION

X-ray microscopy and other applications require high spatial resolution and high efficiency detection of x-rays or high energy particles. In the context of the present invention, high energy particles refer to particles having an energy above 100 eV.

Some current x-ray microscopes utilize optical coupling of a thin scintillator detector to a charge coupled device (CCD) or complementary metal oxide semiconductor (CMOS) camera via an optical microscope. This setup enables high-resolution imaging.

The optical coupling in scintillator-optical microscope-camera detection systems is not perfect, however. The light collection efficiency is limited because of the finite objective numerical aperture (NA) and light loss in the optical microscope. This results in a reduction in Detective Quantum Efficiency (DQE) for the x-ray detection (the so-called quantum sink) and the direct consequence is a reduction in the imaging throughput.

In the typical collection process, scintillator detection systems lose most of the generated light photons. (Most scintillators emit photons in visual light spectrum of 380 nm to 750 nm, but the choices of scintillator wavelength are determined by system needs and availability of suitable scintillators). For example, a 40X objective with NA=0.65 collects about 3% of the light generated by the scintillator and a lower NA objective collects even less. As a rough estimate, the system with a 0.65-NA objective and high efficiency optical and camera system can collect about 40 photon electrons from an x-ray photon of 30 keV and most times these photon electrons are collected in several neighboring pixels. While numerical aperture could be increased to increase collection efficiency this comes at the expense of depth of field, which translates to a lower scintillator thickness and thus X-ray absorption efficiency.

Another option for detecting x-rays or high energy particle beams utilizes semiconductor direct conversion detection materials as described in U.S. Pat. Appl. Publ. Nos. US 2021/0311211 A1 and US 2023/0165541 A1, by Xiaochao Xu and Christoph Graf Vom Hagen. The x-rays or high energy particles create free charge carriers that are directed to a spatial light modulator, such as a liquid crystal (LC) light valve. The electrical charge of the carriers modulate the light valve, which is then illuminated by an external light source of an optical microscope. This configuration can mitigate the loss of light in the optical system over the current scintillator-optical microscope-camera detection systems.

Spectrally/energy-resolving detection systems are also known. Current high-resolution spectrometers for x-ray, γ-ray, high energy and/or charged particles normally consist of a semiconductor (for example, silicon, high-purity germanium at liquid nitrogen temperatures and CdTe/CdZnTe) detector and the accompanying electronics to amplify and process the electrical pulses formed by the impacting individual x-ray photons or high energy particles. Most common ones have only one detector element and therefore only detect the energy and timing information of the incoming x-ray photons or high energy particles.

Pixelated spectrally-resolving detection systems are also known. Commonly, they have repeated identical amplifiers and processing circuits to provide spatial resolution. Such systems, however, generally have lower energy resolution due to the smaller pixels. And, the cost of these pixelated detectors is usually high because the semiconductor processing procedures and high costs of the electronics. A recent development in spatially resolving spectrometer is pnCCD x-ray camera, which provides both high energy and spatial (48 micrometer (µm) pixels) resolution. However, since it can only be made from silicon for now, its applicable detection energies are limited to lower x-ray energies.

A common drawback for all the existing spectrometers is that they can only be used to detect relatively low x-ray photon fluxes. This is because all these detectors need to measure the x-ray photons or high energy particles individually to detect their energies (and positions for spatially resolved ones). Each x-ray photon induces an electronic pulse in the detector and the pulses are measured individually. Higher flux rates thus cause the electronic pulses to overlap each other, therefore leading to pileup problem, which limits the maximum possible flux rate.

For the existing spectrometers (mostly spatially-resolved ones), one way to overcome this pileup problem is to reduce the pixel size. However, there are both physical and technological limits to this strategy. Physically small pixels will have larger crosstalk due to coupling of pixels and more importantly, the weighting potential crosstalk, i.e. the x-ray deposit within one pixel region induces signals at multiple neighboring pixels. Therefore, one quickly reaches the limit where smaller pixels will not significantly reduce the count numbers. Technologically, smaller pixels are also limited by the processing technologies and associated costs.

### SUMMARY OF THE INVENTION

The present invention concerns a spatially-resolved x-ray or other high energy particle detector. Charge carriers are created in an absorption process and these carriers are transferred by an electric field to a light emitting layer, like a light emitting diode (LED), where effective recombination takes place. The present approach optically measures and counts the x-ray photons or high energy particles, typically in a much smaller area. Therefore, they can detect at higher rates and higher spatial resolution. More specifically, the spots are tracked over time and/or dimensionally in order to resolve the energy of the incident x-ray photon/particle, yielding both spectral and spatial resolution. This extends the functionality of the system described in International Publication No. WO 2023/133491 A1, by Philipp Brenner and Xiaochao Xu.

In more detail, in one example, a light emitting layer is coupled with a high-resolution microscope objective. A high speed camera is then used to resolve x-ray or high energy particle interaction events in a very small area. Although the readout of the camera might not be fast, due to the fact of the small area, even when the flux rate is high (e.g., 10⁶ to 10¹² particles mm⁻² s⁻¹), the rate of events impacting individual pixels or regions is low. Therefore, such a setup can detect at a higher flux rate than current x-ray spectrometers. Also with high-resolution detection, spatial resolution can be very high. In addition, there are wide range of choices for the direct-conversion detector materials, and those materials can be tailored for detecting higher energies and/or higher resolution as required by the particular use-case. Further, using direct-conversion detector materials comprising semiconductor detector layer and a light emitting layer allows to omit external light sources and polarization optics.

The present detection systems and associated imaging systems employ a semiconductor detector layer and a light emitting layer such as an LED, in combination with a high speed camera. During operation, incident x-ray photons (or incident high energy particles) will then generate electron-hole pairs (also referred to herein as charge carriers) in the semiconductor detector layer that will then be converted to photons by the light emitting layer. The photons generated by the light emitting layer due to an x-ray or due to a high energy particle will appear as spots. Over time, these spots will exhibit a blooming effect: growing and then dissipating. By detecting the blooming using the camera, both the location and the energy of the incident x-ray photon/ high energy particle can be determined, yielding both spectral and spatial resolution. In general, the spots will bloom very fast, i.e. the time scale of the blooming can even be shorter than the single frame exposure time of a fast camera. So the spots will only show in one frame or a few frames and then disappear in many cases. Nevertheless, useful information can be extracted such as the total number of photons. Using a light emitting layer directly coupled to a semiconductor layer for creating charge carriers based on incident x ray or high energy particles advantageously allows to omit external light sources and polarization optics. On the contrary, light emission and absorption is carried out within the solid-state substrate allowing to control spot formation and blooming by selecting advantageous solid-state properties, such as e.g., effective atomic number Z, electrical resistivity, mobility-lifetime product µτ and thickness of the semiconductor layer. Selecting these properties further allows for ensuring a material time response, i.e., a temporal response of the direct-conversion detector within the order of µs.

In general, according to one aspect, the invention features a spectrally and spatially resolved x-ray and/or high energy (charged) particle detection system. It comprises a detector comprising a semiconductor layer configured for converting photons or particles into charge carriers and a light emitting layer configured for generating light from the charge carriers. An optical microscope and camera are configured to read out the detector. In an embodiment, the optical microscope is configured to receive the light generated from the light emitting layer and the camera is configured to detect the light received via the optical microscope. The detection system further comprises a computer system (controller) that is configured to then obtain images generated by the camera and to track the response of the detector to x-ray photons and/or high energy (charged) particles. Particularly preferred, the camera and/or the computer are configured for an integrated read-out of the detector in order to account for spot formation and blooming events on short timescales, due to the material response time in the order of µs.

In some examples, the semiconductor layer can be amorphous selenium (a-Se), GaAs, CdZnTe, CdTe, or perovskite crystal (ABX3). The light emitting layer can be an organic light emitting diode (OLED), GaAs, AlGaAs, InGaAs, CdTe or CdZnTe. In a preferred embodiment, the semiconductor layer and the light emitting layer are formed of the same material (i.e., the detector structure is not necessarily a heterostructure). Further preferred, the semiconductor layer and the light emitting layer are based on the same substrate, wherein the light emitting layer is a ternary alloy of the semiconductor layer, which facilitates the fabrication of the device and improves light extraction from the detector (hetero)structure.

Further preferred, the material of the LED layer and/or the material of the semiconductor layer are selected such that a maximum energy of light emitted by the LED layer is less than the bandgap of the semiconductor layer. By choosing the material in such manner it can be advantageously ensured that the light emitted by the LED layer is preferably not or only weakly absorbed by the semiconductor layer. IN other words, the material is selected such that the degree of reabsorption in the semiconductor layer is so low that a feedback loop gain between LED layer and semiconductor layer is less than 1 and positive feedback can be avoided.

The computer system will often track the blooming events in the detector induced by the x-ray photons or high energy (charged) particles to resolve locations on the detector and energy of the x-ray photons or charged particles. The computer system might determine the energy of the x-ray photons or charged particles by reference to an energy/intensity map that relates a maximum spot intensity to an energy of the received x-ray photons or charged particle.

In general, according to another aspect, the invention features a particle detection method comprising converting x-ray photons and/or charged particles into electron-hole pairs (as charge carriers) in a detector comprising a semiconductor layer for converting (x ray) photons or high energy particles into charge carriers (the semiconductor layer creating charge carriers if the energy of the (x ray) photons or high energy particles exceeds at least the band gap of the semiconductor layer) and converting one of the charge carrier types generated by the semiconductor layer into photons (preferably photons of visible light or IR, i.e., photons with an energy below the energy of the influx radiation or particles) by a light emitting layer of the detector, the light emitting layer being configured for generating light (preferably visible or IR light, see above) from the charge carriers (i.e., one of the electron-hole pairs), reading out the detector (particularly the photons emitted from the detector) with a camera coupled to the detector, and processing images generated by the camera and tracking the response of the detector to x-ray photons or high energy (charged) particles over time to determine the position and the energy of the x-ray photons or high energy (charged) particles.

In general, according to still another aspect, the invention features an imaging system. This system comprises an object stage system configured for holding an object, a detector, including a semiconductor layer configured for converting high energy (x ray) photons or high energy particles into charge carriers and a light emitting layer configured for generating light from the charge carriers for detecting high energy (x-ray) photons or high energy (charged) particles from the object, a camera coupled to the detector by an optical microscope, and a computer system that is configured to obtain the images generated by the camera and to track the response of the detector to x-ray photons or charged particles over time to image the object and to determine an energy of the x-ray photons or charged particles.

In general, according to another aspect, the invention features a method for calibrating an x-ray photon or charged particle detection system. The method comprises generating high energy (x-ray photons) or high energy (charged) particles of known energy, converting these high energy photons or particles into electron-hole pairs (as charge carrier types) in (the semiconductor layer of) a detector including a (the) semiconductor layer configured for converting high energy (x ray) photons or high energy particles into charge carriers and converting one of the charge carrier types into photons in a light emitting layer of the detector, the light emitting layer being configured for generating light, preferably visible or IR light, from the charge carriers. The detector (particularly the photons emitted therefrom) is then readout with a camera and images generated by the camera are processed to track spots generated by the x-ray photons or charged particles received by the detector for determining a relationship between the spots and the energy of the x-ray photons or charged particles.

The above and other features of the invention including various novel details of construction and combinations of parts, and other advantages, will now be more particularly described with reference to the accompanying drawings and pointed out in the claims. It will be understood that the particular method and device embodying the invention are shown by way of illustration and not as a limitation of the invention. The principles and features of this invention may be employed in various and numerous embodiments without departing from the scope of the invention.

### BRIEF DESCRIPTION OF THE DRAWINGS

In the accompanying drawings, reference characters refer to the same parts throughout the different views. The drawings are not necessarily to scale; emphasis has instead been placed upon illustrating the principles of the invention. Of the drawings:
Fig. 1A and 1B are a side view showing a detector for x-ray or high energy particles with a semiconductor - light emitting diode (LED) detector structure;
Fig. 2 is a side schematic perspective view showing the layers of an exemplary a GaAs detector/LED device;
Fig. 3 shows a simulated GaAs detector/LED device band diagram at a forward bias of V= 5 V; *E_{c}* is the conduction band, *Eᵥ* valence band; *E_{fn}* is the quasi-Fermi level of electrons and *E_{fy}* is the quasi-Fermi level of holes; and the right diagram is a blow up of the LED section of the device;
Fig. 4A shows the simulated electron and hole concentrations of the GaAs detector/LED device at forward bias of 5V as a function of distance; and Fig. 4B shows simulated rates of various carrier recombination processes near the active QW region as function of distance;
Fig. 5 is a plot of IQE at different bias voltages for the simulated GaAs detector/LED device;
Fig. 6 shows the GaAs Detector/LED simulated rates at forward bias of V= 5 V with illumination at z=0; The right plot is a blow up around the active QW region of the device; the light intensity corresponds to relative light intensity of 0.1 as in Figs. 7A and 7B;
Fig. 7A is a simulated total current output of the GaAs detector/LED device with different light intensity at forward bias of 5 V. Fig. 7B is a simulated IQE of the GaAs detector/LED device with different light intensity at forward bias of 5 V;
Fig. 8 is a schematic side view showing the layers of an embodiment of a CdTe detector/LED device;
Fig. 9 shows the simulated CdTe detector/LED device band diagram at forward bias V= 5 V. *E_{c}* is the conduction band, *Eᵥ* valence band. *E_{fn}* is the quasi-Fermi level of electrons and *E_{fy}* is the quasi-Fermi level of holes, the right diagram is a blow up of the LED section of the device;
Fig. 10A shows the simulated electron and hole concentrations of the CdTe detector/LED device at forward bias of 5 V as a function of distance; and Fig. 10B shows simulated rates of various carrier recombination processes near the active QW region as function of distance;
Fig. 11 shows the CdTe Detector/LED simulated rates at forward bias of V= 5 V with illumination at z=0, the right diagram is a blow up around the active QW region of the device, the light intensity corresponds to relative light intensity of 0.1 as in Figs. 12A and 12B;
Fig. 12A shows the simulated total current output of the CdTe detector/LED device with different light intensity at forward bias of 5 V; and Fig. 12B shows the simulated IQE of the CdTe detector/LED device with different light intensity at forward bias of 5 V;
Fig. 13 is a schematic side view of a semiconductor light emitting x-ray detector using a perovskite semiconductor and an OLED;
Fig. 14 shows the semiconductor LED x-ray detector with its optical readout;
Fig. 15 is a schematic diagram of an x-ray microscope to which the present invention is applicable;
Fig. 16 is a schematic diagram showing spot blooming associated with various detected x-rays or high energy particles across the extent of the semiconductor detector layer 74;
Fig. 17 is a flow diagram showing a method of calibrating the detection system; and
Fig. 18 is a flow diagram showing method of generating an energy resolved image or projection using the detection system 100.

### DETAILED DESCRIPTION OF THE PREFERRED EMBODIMENTS

The invention now will be described more fully hereinafter with reference to the accompanying drawings, in which illustrative embodiments of the invention are shown. This invention may, however, be embodied in many different forms and should not be construed as limited to the embodiments set forth herein; rather, these embodiments are provided so that this disclosure will be thorough and complete, and will fully convey the scope of the invention to those skilled in the art.

As used herein, the term "and/or" includes any and all combinations of one or more of the associated listed items. Also, all conjunctions used are to be understood in the most inclusive sense possible. Thus, the word "or" should be understood as having the definition of a logical "or" rather than that of a logical "exclusive or" unless the context clearly necessitates otherwise. Further, the singular forms and the articles "a", "an" and "the" are intended to include the plural forms as well, unless expressly stated otherwise. It will be further understood that the terms: includes, comprises, including and/or comprising, when used in this specification, specify the presence of stated features, integers, steps, operations, elements, and/or components, but do not preclude the presence or addition of one or more other features, integers, steps, operations, elements, components, and/or groups thereof. Further, it will be understood that when an element, including component or subsystem, is referred to and/or shown as being connected or coupled to another element, it can be directly connected or coupled to the other element or intervening elements may be present.

It will be understood that although terms such as "first" and "second" are used herein to describe various elements, these elements should not be limited by these terms. These terms are only used to distinguish one element from another element. Thus, an element discussed below could be termed a second element, and similarly, a second element may be termed a first element without departing from the teachings of the present invention.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

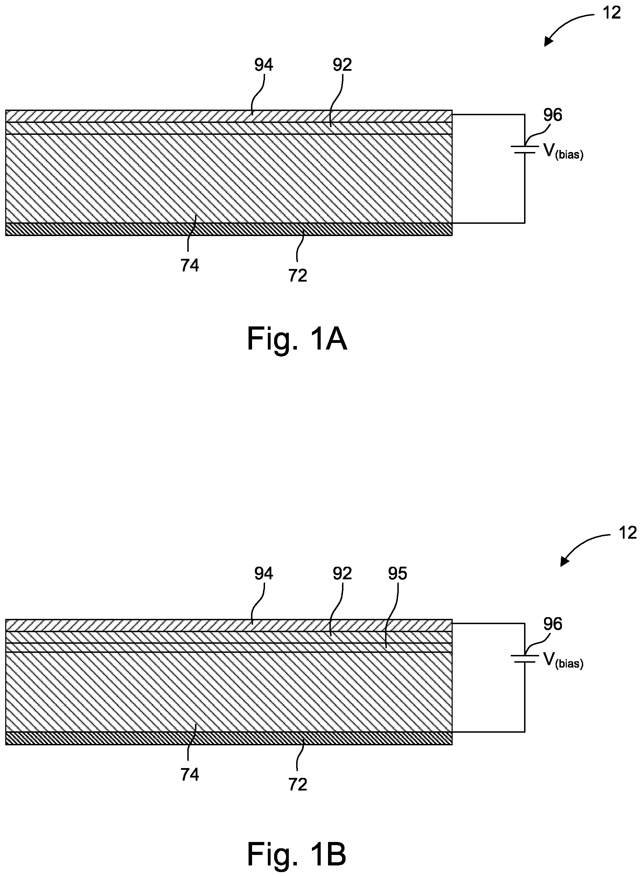

Fig. 1A shows the basic arrangement of semiconductor light emitting diode (LED) detector for x-ray or high energy particles 12 for use in an imaging spectrometer.

The detector 12 comprises LED layer 92 disposed on one side of a semiconductor detector layer 74. An x-ray-side electrode layer 72 (also referred to as radiation influx side layer 72) is deposited on an (opposite) x-ray side (or radiation influx side) of the semiconductor detector layer 74. On an optical-side (also referred to as LED light outflux side) of the semiconductor detector layer 74 is the LED layer 92 followed by an optical-side electrode layer 94 such as a transparent Indium Tin Oxide (ITO).

The layer 74 is preferably a semiconductor having a relatively high effective atomic number Z and density to effectively stop and absorb the x-ray radiation and in order to spatially confine created charge carriers within the semiconductor layer 74. The electrical resistivity should preferably be high with a value of larger than 10⁶ Ω cm more preferred larger than 10⁹ Ω cm preferred, and most preferred larger than 10¹¹ Ω cm, in order to reduce the dark current and in order to spatially confine created charge carriers within the substrate material. In addition, the thickness of the semiconductor is chosen such that at least 5%, preferably more than 50%, particularly preferred more than 90% of one type of the excited charge carriers (electrons or holes of the electron-hole pairs) can be transported through the thickness of the semiconductor layer 74 before it recombines in the absorption layer, i.e. the excited charge carrier that is used for injection into the LED layer 92 has a relatively high mobility-lifetime product *µτ*. This is one property of a semiconductor material, in which µ can be *µₑ* or *µₕ*, the mobility of the either electrons or holes; and τ is the corresponding *τₑ* or *τₕ,* the lifetime of the electrons or holes. Larger *µτ* is preferred for better detector performance. Preferred *µτ* values can be determined from the charge collection Hecht equation $C \left(V\right) = \frac{μτV}{{d}^{2}} \left(1-{e}^{\frac{{d}^{2}}{μτV}}\right)$, where C is the charge collection efficiency of one carrier species (electron or hole) , and it should at least 5%, preferably more than 50%, particularly preferred more than 90%; V is the detector DC bias voltage determined by the detector requirements, such as maximum breakdown voltage, safety margin etc., and practical considerations. It can range from a few volts to order of kilovolt; d is the thickness of the layer 74 (see next paragraph). Other requirements are similar to those required with other semiconductor x-ray detectors, such as low polarization and stability over time and other conditions. In other words, the combination of a relatively high effective atomic number Z and density, electrical resistivity of larger than 10⁶ Ω cm, a relatively high mobility-lifetime product *µτ* and the thickness of the semiconductor layer 74 ensures that the charge carriers reach the LED layer 92 in a spatially confined manner, without fanning out to an extent that a spatial resolution of the detector would suffer. Further, these parameters are chosen such that a material time response in the order of µs or shorter can be ensured.

The semiconductor layer 74 will preferably have a thickness in the range of about one micrometers to a few mm, preferably a thickness between 1 micrometer to 1 mm, further preferred between 5 and 500 micrometers. In some preferred examples, this layer is (comprises or consists of) amorphous selenium (a-Se), GaAs, CdZnTe, CdTe, or crystalline and amorphous perovskite semiconductor crystal materials (ABX3).

The working mechanism is as follows. A charge cloud (cloud of electron-hole pairs) is created within the thick semiconductor layer 74 by absorption of the x-ray photon or high energy particle. The electron and holes travel in opposite directions due to the applied electric field though the x-ray-side electrode layer 72 and the optical-side electrode layer 94 by bias voltage source 96. One of the charge carrier types is injected into a thin emission zone of the LED layer 92. Its structure is tailored for effective radiative recombination. One example of the tailored design can be found at Li N, Han K, Spratt W, Bedell S, Ott J, Hopstaken M, et al. Ultra-low-power sub-photon-voltage high-efficiency light-emitting diodes. Nature Photonics. 2019; 13(9):588-92. The radiative recombination results in the emission of a photon, which propagates through the optical-side electrode layer 94 and can then be detected in a similar manner like a thin scintillator. The light emitting layer 92 preferably is an inorganic or organic LED.

An important requirement for the LED layer 92 is that it operates efficiently over a wide range of charge carriers that are injected into the layer. The amount of charge carriers injected depends on the amount of absorbed X-ray photons or high energy particles and thus varies throughout operation. Special layer stacks and growth conditions must be considered in order to ensure efficient operation at low charge carrier injection densities. See Li, et al.

Advantages exist when organic light emitting diode (OLED) layer is used for the LED layer 92. OLEDs exhibit high efficiencies at low charge carrier injection densities while an efficiency roll-off is usually observed at high charge injection densities. Another advantage of using an OLED is that the layers can be deposited by thermal evaporation on different absorber substrates as no lattice matching has to be taken into account as it is the case for epitaxial techniques which must be applied for inorganic semiconductors.

In one preferred embodiment, the detector 12 uses a GaAs based semiconductor detector absorber layer 74. For the LED layer 92, its related alloys, preferably ternary allows, AlGaAs and InGaAs are used to form a heterostructure. One advantage of GaAs is that it has one of the largest radiative recombination rates among commonly available semiconductor materials. In addition, because of its wide availability, the technologies for making such LEDs are mature and easily available.

The LED based detector will cover a wide range of x-ray flux levels (e.g., 10⁴ to 10¹² particles mm⁻² s⁻¹), when the flux level is low, the x-ray generated current in the detector is rather small. A typical LED has a rather low light efficiency when the driving current is low because non-radiative recombination overtakes radiative recombination at low charge injection rate.

In one preferred example, the LED layer 92 is designed for high efficiency at ultra-low current to overcome the shortcomings of regular LEDs that designed to work at higher injection currents. A single quantum well (QW) is used with a specially designed well and cladding, in one embodiment. The improvements are achieved via two mechanisms: (1) a high-quality InGaAs/InGaP or GaAs/InGaP interface reduces the interface recombination velocity (IRV) and (2) large valence band offset ${Q}_{v} = \frac{Δ {E}_{v}}{\left(Δ{E}_{v}+Δ{E}_{c}\right)}$ to make hole density *p* much larger than electron density n within the QW (or large conduction band offset ${Q}_{c} = \frac{Δ {E}_{c}}{\left(Δ{E}_{v}+Δ{E}_{c}\right)}$ to make electron density *n* much larger than hole density *p* within the QW). See Li, et al.

In another example, the LED layer 92 has an InGaP/GaAs/InGaP double heterojunction, which have been demonstrated for high quantum efficiency. See Li, et al. In one specific preferred example, the InGaP band gap is 1.90 eV, and the InGaP/GaAs conduction band offset is 0.10 eV and valence band offset 0.38 eV.

Fig. 1B shows another embodiment that adds a light reflecting layer such as a Bragg distributed reflector layers.

In more detail, it should be noted that half of the light from the active layer of the LED layer 92 goes toward the semiconductor layer 74 instead of the optical-side electrode layer 94. Thus, to further increase the output of the light, a distributed Bragg reflector (DBR) or other light reflecting layer 95 is added between the LED layer 92 and the semiconductor layer 74 to reflect most of the light towards the optical-side electrode layer 94 and the output aperture. This is similar to the DBRs in vertical-cavity surface-emitting lasers (VCSEL) for example. The Bragg layer advantageously allows to avoid back-illumination of light created in the LED layer 92 into the semiconductor layer 74 and thus any downstream charge carrier creation based on the back-illuminated light , which could lead to blurring of the formed spots due to spatially unconfined photon generation.

In addition, to further shape the wavefront of the emission light, the detector includes a dielectric material layer on the optical-side, particularly a dielectric material layer can be deposited onto the output window or the transparent electrode, or the transparent electrode can be patterned. Potentially, the shaped wavefront together with the collection optics can improve the light collection efficiency. In addition, antireflective (AR) layers and/or metalenses can be added.

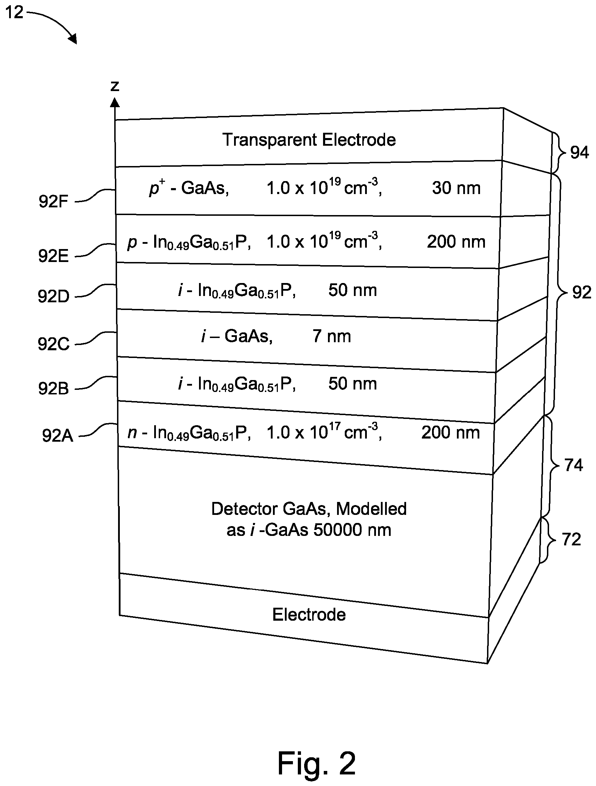

Fig. 2 shows one specific embodiment of the detector 12.

The semiconductor detector absorber layer 74 is a thick GaAs layer for detecting x-ray. The thick GaAs layer is typically between 0.1 to over 100 microns thick. (Note, however, for simulation purposes, a thickness of 5 µm was used.) It is located on the x-ray-side electrode layer 72. The LED heterostructure layer 92 includes an n type In_{0.49}Ga_{0.51}P contact layer 92A of 200 nanometers (nm), followed by an In_{0.49}Ga_{0.51}P layer 92B of 50 nm, a GaAs layer 92C layer of 7 nm, and another In_{0.49}Ga_{0.51}P layer 92D of 50 nanometers to form a double heterojunction. A p type In_{0.40}Ga_{0.51}As hole contact layer 92E of 200 nm thickness and a p+ GaAs hole contact layer 92F of 30 nm thickness are used before the optical-side electrode layer 94.

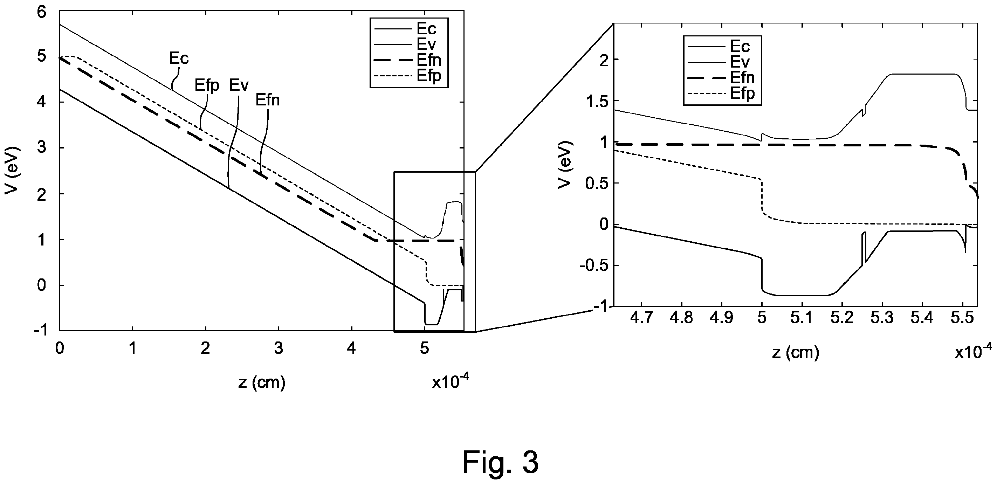

Fig. 3 shows the simulation results of the band diagram at a bias voltage of 5 V. The electric field (proportional to the gradient of *E_{c}* and *Eᵥ*) within bulk of this layer is uniform. Therefore, the device should behave similarly if the bias voltage is varied to keep the electric field equal with the increase of thickness of the GaAs layer. This GaAs layer can be a semi-insulating GaAs wafer or chromium compensated GaAs (GaAs:Cr) wafer. The simulation included the high resistivity of an idealized intrinsic GaAs layer. The contacts were assumed to be ohmic. Quantization effect on band structure were included.

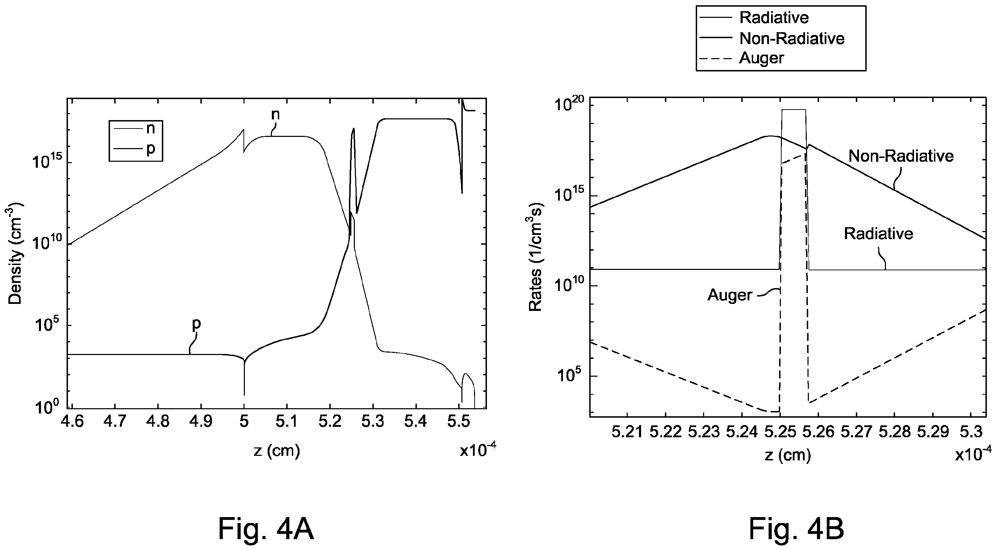

Fig. 4A shows simulated electron and hole concentrations at a bias voltage of 5 V. The hole concentration is much larger than electron concentration at the active QW region.

Fig. 4B shows the rates of different carrier recombination processes. At the active QW region, the radiative rate is much higher than other processes. This indicates a rather high internal quantum efficiency (IQE).

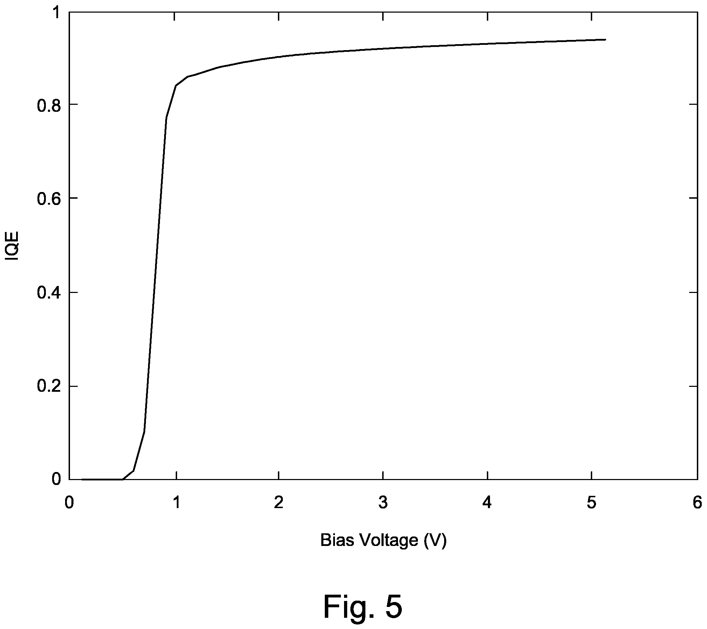

As shown in Fig. 5, when varying the forward bias voltage, the IQE is kept rather high when the bias voltage is more than about ~1 V.

The above simulations were done without external x-ray, high energy particles or light to generate carriers in the detector region. To detect a signal, x-ray is illuminated onto the detector side to generate extra carriers. To simulate the extra carriers, a solar cell setting was employed to generate carriers within the detector region. The solar cell setting simulates illumination with infrared, visible or UV light. The difference between illuminating with visible light and x-ray is the distribution of the extra carriers, i.e., the electron-hole pairs, in the semiconductor layer. With x-ray, the charge carriers spread out across the thickness of the detector, while with visible light, the charge carriers concentrate near the front surface of the detector, due to the lower penetration depth of the lower energy influx radiation. After the carriers are generated, they drift towards the LED heterostructure 92.

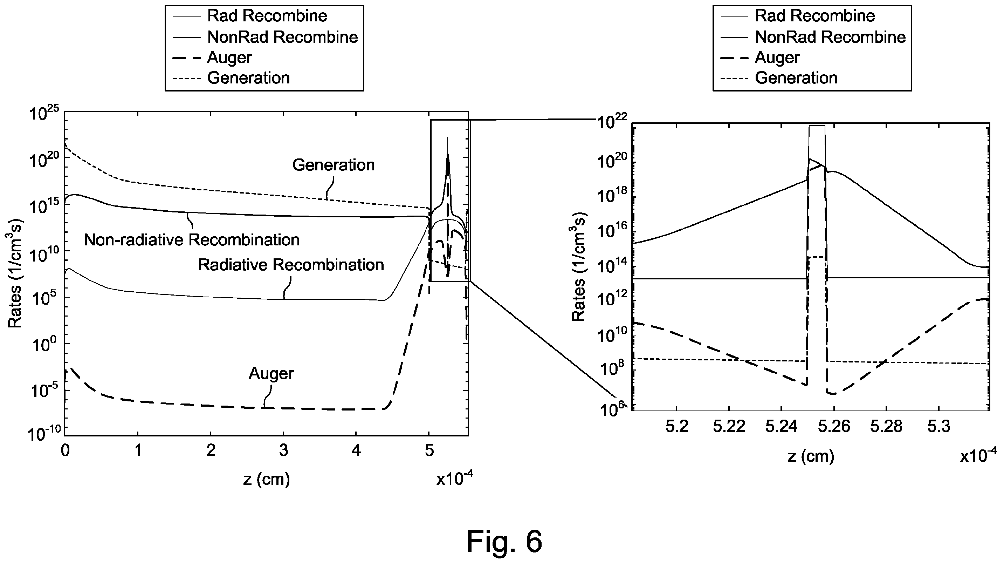

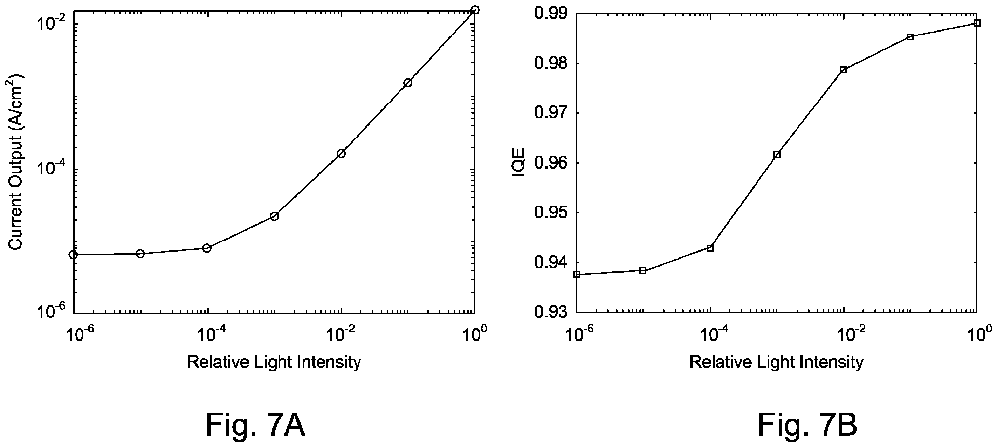

Fig. 6 shows rates of various processes, including the recombination rates and generation rate at a light intensity of 0.1 (relative to an arbitrary intensity as shown in Figs. 7A and 7B). The generation was highest at z =0 as that was where the light entered the device. The relative positions of the 3 recombination rates were similar to those when no light was on. However, the absolute values were much larger because many extra carriers were generated at the z=0 and drifted to the LED. We also see a generation rate at the LED on this logarithm scale plot because there was small amount of the light penetrating the LED layers.

In the simulation, Fig. 7A shows the total current for the illumination light intensity to obtain the response of the device. As set forth above, in the simulated solar cell setup, the illumination light is infrared, visible or UV light The response was linear. However, there was a small offset due to the intrinsic conductivity of the GaAs detector layer. Fig. 7B shows that there was a small increase in IQE as light intensity increased, which can cause a small nonlinearity in the output light response to the light intensity of the radiation influx from the radiation influx side. However, the change is rather small over a few decades of light intensity change, therefore the nonlinearity is small, making calibration easy.

The simulations showed that the above design of the detector/LED worked well with high IQE (>90%) at all light/x-ray exposure conditions. The biggest assumption is that the detector GaAs behaved like a pure intrinsic GaAs and the major trapping effects were ignored by the compensated trapping centers.

In another embodiment, the detector 12 uses single-crystalline CdTe or CdZnTe semiconductor detector layer 74 and CdTe or CdZnTe LED heterostructure 92. CdTe and CdZnTe has been studied for many years for x-ray and γ-ray detectors and they are two most widely used room-temperature semiconductor detector materials. They have larger mean atomic numbers than GaAs, therefore better stopping power than GaAs. And this makes them especially suitable for x-rays with energy higher than 10 keV.

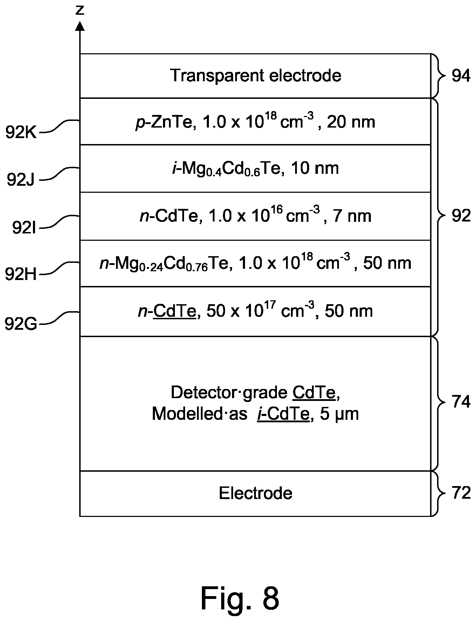

Fig. 8 shows a semiconductor LED x-ray detector using a CdTe material system for use in an imaging spectrometer.

The semiconductor detector layer 74 is a thick CdTe layer for detecting x-ray. The thick CdTe layer can be up to a few hundred microns thick. (Note, however, for simulation and illustration purposes, it is 5 µm.) It is located on the x-ray-side electrode layer 72.

The LED heterostructure layer 92 includes an n type CdTe layer dopant concentration 5.0 × 10¹⁷ *cm*⁻³, 92G of 50 nm thick, followed by an *n*-Mg_{0.24}Cd_{0.76}Te, 1.0 × 10¹⁸ *cm*⁻³, barrier layer 92H of 50 nm thick, followed by an n-CdTe, 1.0 × 10¹⁶ *cm*⁻³, active quantum well layer 92I of 7nm thick, followed by an *i-*Mg_{0.4}Cd_{0.6}Te 10 nm barrier layer 92J, followed by a p-ZnTe, 1.0×10¹⁸ *cm*⁻³, contact layer 92K of 20 nm thick. The contact layer makes an electrical connection to the optical-side electrode layer 94.

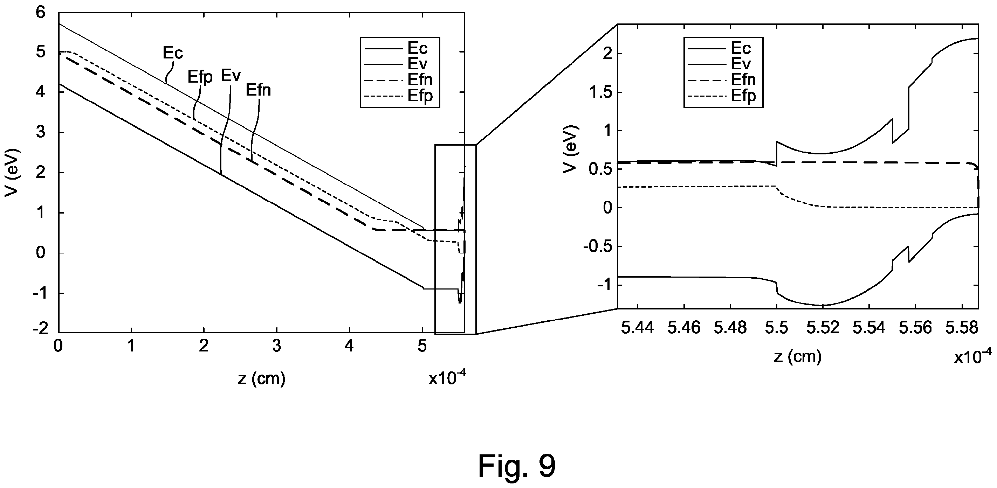

Fig. 9 shows the band diagram of the CdTe LED without external illumination at a bias voltage of 5 V. For the CdTe LED, at the QW region, the electron well was deeper than the hole well. And again, the electric field was constant in the bulk of the detector region.

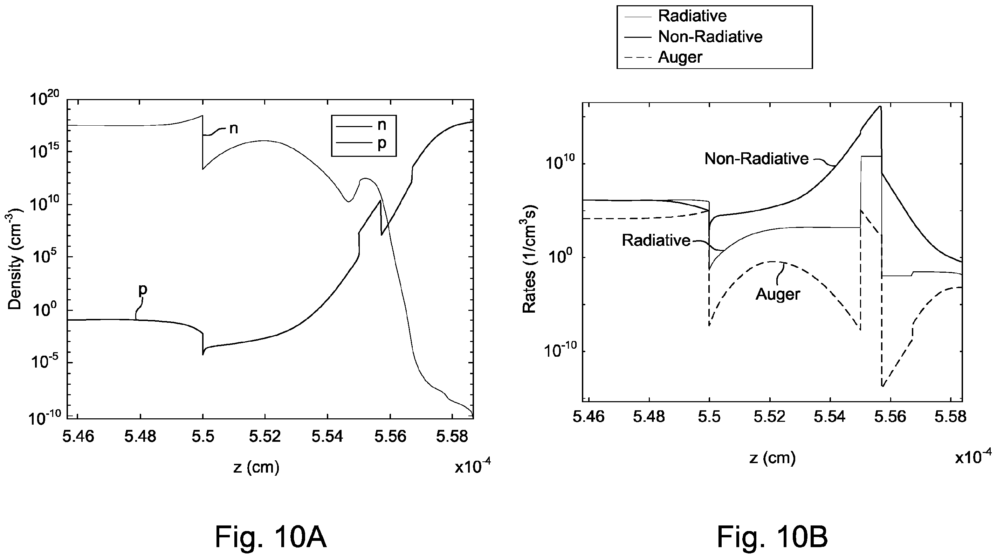

Fig. 10A shows the simulated electron and hole concentrations of the CdTe device at forward bias of 5V. At the active region, the electron concentration is much larger than the hole concentration. Fig. 10B shows the rates of different carrier recombination processes. At the active QW region, the non-radiative rate is much higher than other processes. This indicates that at 5V bias and no external illumination, the device will almost not emit light. This is not a problem for the detector because with external illumination, the device emits light.

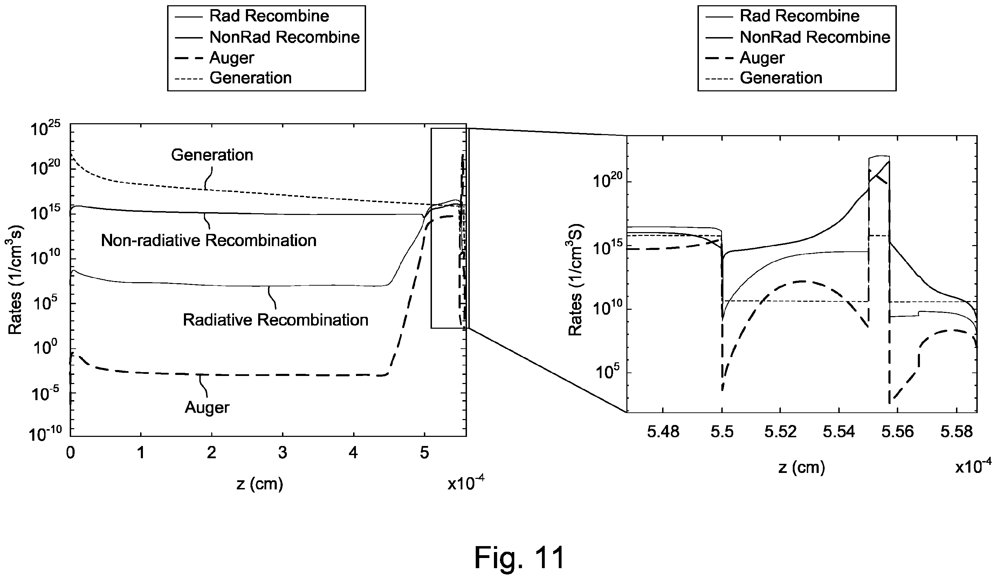

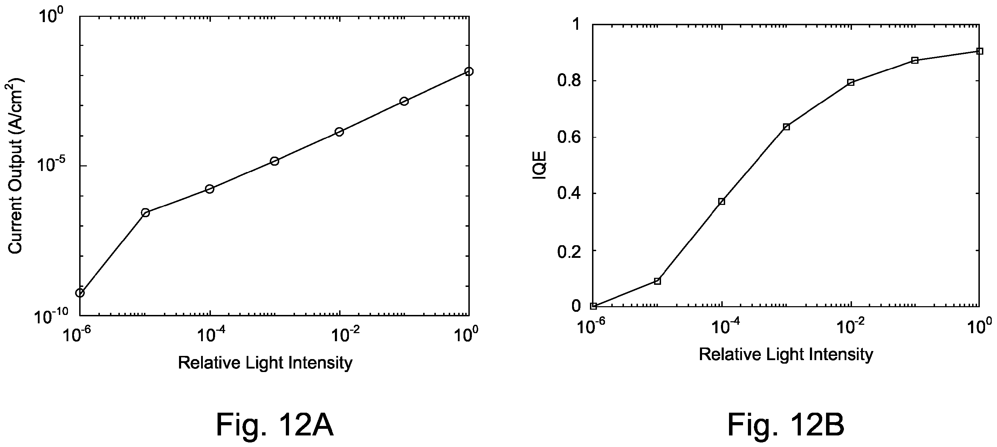

Fig. 11 shows rates of various processes, including the recombination rates and generation rate at a relative light intensity of 0.1 as in Figs. 12A and 12B. With carriers created (injected) due to the external illumination, the radiative recombination rate increased.

The illumination light intensity on the radiation influx side was varied to obtain the response of the device. Fig. 12A shows the total current. Fig. 12B shows the IQE. IQE changes from 0 to around 0.9 at highest illumination. This will cause nonlinearity in the response. However, this simulation covered 6 orders of magnitude. In addition, the nonlinearity can be calibrated. Lastly, the simulations treated the CdTe x-ray layer as an intrinsic semiconductor therefore carried a very low dark current at zero external illumination. The actual dark current is normally a few order magnitudes larger than the intrinsic value, this current will increase the IQE and make the IQE varies less over the different light intensities.

In another embodiment, the detector uses a perovskite semiconductor with the chemical formula ABX₃, such as CsPbBr₃, as an absorption semiconductor layer and an organic layer stack as LED. Similar to the inorganic LED device, the organic layer stack has several different layers for charge transport, charge injection or charge blocking around the emission layer. The emission layer itself includes different types of organic molecules, preferentially a phosphorescence emitter, such as Ir(ppy)₃ or a molecule allowing for thermally activated delayed fluorescence (TADF), such as 4CzIPN. OLEDs with the latter material have to be proven to exhibit a high IQE and a good linear response at different current densities, as well as a moderate lifetime of the excited state in the emitter molecule.

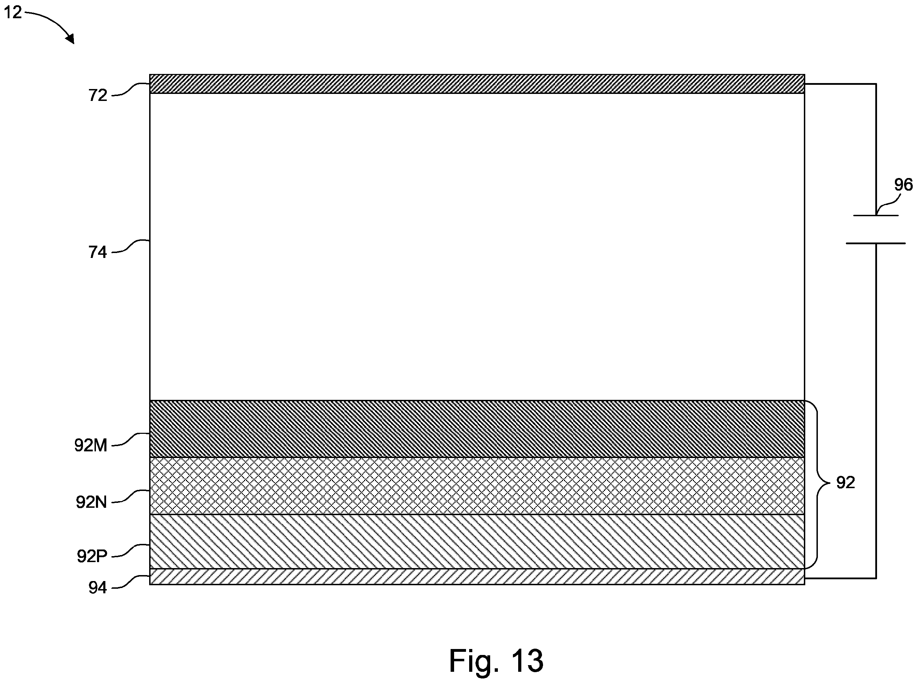

Fig. 13 shows a detector 12 using a combination of the perovskite absorbers and the organic layer stack for use in an imaging spectrometer.

The detector 12 comprises LED layer 92 disposed on one side of a CsPbBr₃ semiconductor detector layer 74, which can be about 50 micrometers thick. A gold x-ray-side electrode layer 72 is deposited on an x-ray side of the semiconductor detector layer 74. On an optical-side of the semiconductor detector layer 74, is the OLED layer 92 followed by the transparent optical-side electrode layer 94 of indium tin oxide, for example.

OLED layer 92 include a TpBi layer 92M of about 65 nm, followed by a 4CziPN layer 92N of about 15nm, followed by an Alpha-NPD layer 92P of about 35 nm.

To collect as much as possible light emitted from the LED (for both the inorganic LED or OLED), a few of the following measures can be taken separately or together:

When an air objective is used to collect the light, the highest possible NA should be employed for the selected magnification. However, with air objective, a lot of light will be lost. With a GaAs LED, the index of GaAs is about 3.6 at around 850nm. The index mismatch with air is large. Without any other measures, most light will be trapped in the emission layer.

It is preferred that a single layer or multiple layers of anti-reflective coating is applied onto the LED active layer. The transparent electrode material, ITO has an index close to the optimal index of 1.9 at 850 nm and can act as an antireflective coating with proper ITO receipt to fine tune the exact composition and processing to best match the anti-reflective conditions.

To further increase the NA of the objective, an immersion-type objective is preferred. The coupling of the LED with the objective can be a liquid, or an optical glue that fixes the LED with the objective. Because the objective focus onto the very thin LED active layer, no further adjustment is needed after it is focused, the glue option may be a preferred way in most cases.

A metalens may be used to replace expensive and bulky high-NA objective for light collection.

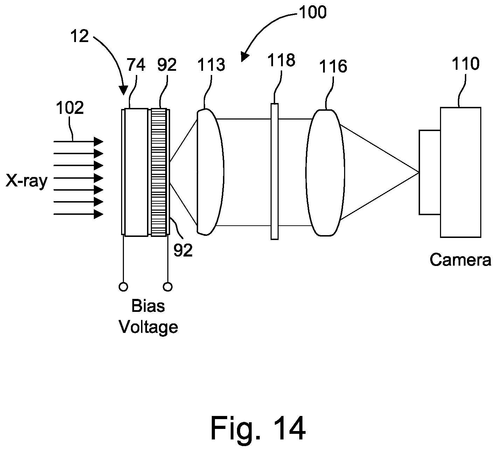

Fig. 14 shows a light optical microscope-based x-ray detection system 100 employing the detector 12.

In more detail, the x-ray detection systems 100 generally comprises the semiconductor LED x-ray detector 12. Incoming x-rays or high energy (charged) particle beam 102 are received in the semiconductor layer 74. The resulting electric charges are injected into LED layer 92.

The light generated by the LED layer 92 is collected by objective lens 113. A tube lens 116 provides the light to the camera 110. Another element 118 can be added in the infinity space to achieve other optional functions. For example, an x-ray shielding window to protect the tube lens 116 and the camera 110.

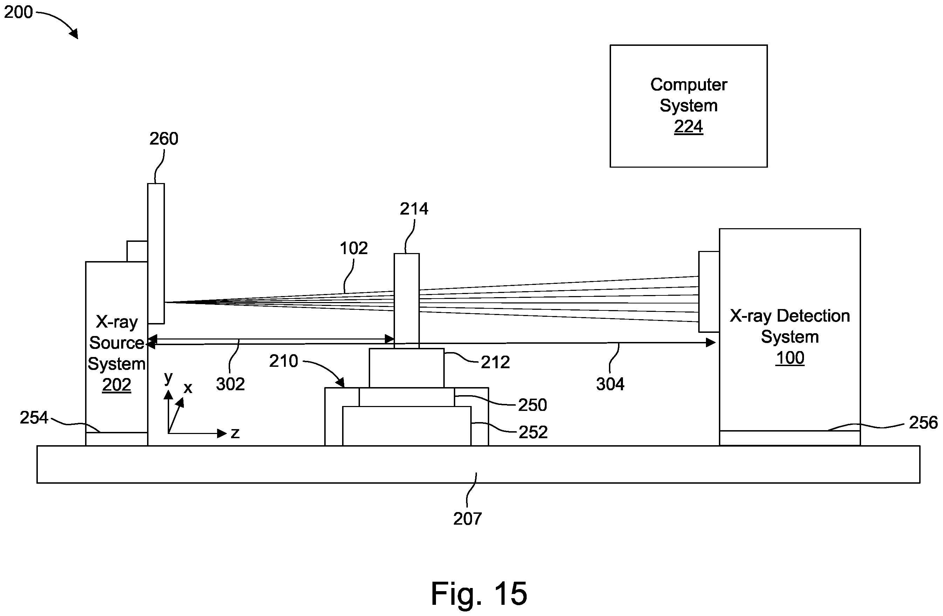

For context, Fig. 15 is a schematic diagram of an X-ray CT microscopy system 200 to which the x-ray detection system 100 and its semiconductor LED x-ray detector 12 are applicable.

Nevertheless, the present invention is applicable to charged particle analysis systems and non-microscopy systems.

The microscope 200 generally includes an X-ray imaging system that has an X-ray source system 202 that generates a polychromatic that is then filtered or possibly monochromatic X-ray beam 102 and an object stage system 210 with object holder 212 for holding an object 214 and positioning it to enable scanning of the object 214 in the stationary beam 102. The x-ray detection system 100 detects the beam 102 after it has been modulated by the object 214. A base such as a platform or optics table 207 provides a stable foundation for the microscope 200.

**In** general, the object stage system 210 has the ability to position and rotate the object 214 in the beam 102. Thus, the object stage system 210 will typically include a precision 3-axis stage 250 that translates and positions the object along the x, y, and z axes, very precisely but over relatively small ranges of travel. This allows a region of interest of the object 214 to be located within the beam 102. The 3-stage stage 250 is mounted on a rotation stage 252 that rotates the object 214 in the beam around the y-axis. The rotation stage 252 is in turn mounted on the base 107.

The source system 102 will typically be either a tunable monogenetic synchrotron x-ray radiation source or alternatively a set of radioisotopes with predefined energy peaks that are within the detection energy ranges, i.e., the energy ranges in which the detector functions for resolving energies. **In** still other examples, a filtered "laboratory x-ray source" is used to generate monochromatic or highly monochromatic x-rays.

The x-ray beam generated by source 202 is preferably conditioned to suppress unwanted energies or wavelengths of radiation especially when using the laboratory x-ray source. For example, undesired wavelengths present in the beam are eliminated or attenuated, using, for instance, energy filters (designed to select a desired x-ray wavelength range (bandwidth)) held in a filter wheel 260. Conditioning is also often provided by collimators or condensers and/or an x-ray lens such as a zone plate lens.

When the object 214 is exposed to the X-ray beam 102, the X-ray photons transmitted through the object form a modulated x-ray beam that is received by the detection system 100. In some other examples, a zone plate objective x-ray lens is used to form an image onto x-ray detection system 100.

Typically, a magnified projection image of the object 214 is formed on the detection system 100. The magnification is equal to the inverse ratio of the source-to-object distance 302 and the source-to-detector distance 304.

Typically, the x-ray source system 202 and the detection system 100 are mounted on respective z-axis stages. For example, in the illustrated example, the x-ray source system 202 is mounted to the base 207 via a source stage 254, and the detection system 100 is mounted to the base 207 via a detector stage 256. **In** practice, the source stage 254 and the detector stage 256 are lower precision, high travel range stages that allow the x-ray source system 202 and detection system 100 to be moved into position, often very close to the object during object scanning and then be retracted to allow the object to be removed from, a new object to be loaded onto, and/or the object to be repositioned on the object stage system 210.

The operation of the system 200 and the scanning of the object 214 is controlled by a computer system 224 that often includes an image processor subsystem, a controller subsystem. The computer system is used to readout the optical image detected by the camera 110 of the detection system 100. The computer system 224, with the possible assistance of its image processor, accepts the set of images from the detection system 100 associated with each rotation angle of the object 214 to build up the scan.

According to the invention, the detection system 100 generally comprises the detector 12 and the camera 110. The camera is preferably a high speed camera in the sense that it captures images at a rate that is fast enough to detect the blooming induced in the detector 12 associated with the detection of individual x-ray photons or other particles. As a result, the camera will typically capture images at greater than 60 frames a second. It preferably has a speed of equal to or greater than 120 frames per second as is preferably faster than 500 or even a 1000 frames per second or faster.

The operation of the system 200 and the scanning of the object 214 is controlled by a computer system 224 that often includes an image processor subsystem and a controller subsystem. The computer system is used to set bias voltages of the detector 12 and to readout the optical images detected by the camera 110 of the detection system 100.

According to the invention, the computer system 224 obtains the images generated by the camera 110 and tracks blooming spots induced by the x-ray photons or high energy (charged) particles detected in the detector 12 over time, both the location on the detector 12 and the energy of the photons can be determined, yielding both spectral and spatial resolution by tracking the light photons generated by the LED layer 92.

Specifically, the computer system 224 determines the energy of the photons or other particles by reference to an energy/intensity map 310 such as a lookup table (LUT). This map relates the maximum spot intensity or integrated spot intensity, or a fit to the intensity map to the energy of the received particle. Preferably, an integrated readout is implemented to measure the total light output during one spot blooming event. This advantageously accounts for more dynamic spot formation in the LED structure, particularly when compared to using slower-responding optical valve methods.

It should be noted that the computer system need not be a unitary device. For example, a single-board computer or microcontroller might be used to as the control system for the microscopy system 200. A separate computer might be used to process the images generated by the camera to generate the spatially and spectrally resolved object images or projections of the object and/or perform tomographic reconstruction of the object based on the various projections. In fact, the camera images might be stored and then later processed or reprocess in order to generate the object images and reconstructions. As a result, a special purpose computer such as a graphic processing unit (GPU), application specific integrated circuit (ASIC), field programmable array (FPGA), general purpose computer or some combination of these or other computer systems would be included as part of the computer system 224 to process the images. In addition, these computer systems could also be integrated within the housing of the camera 110.

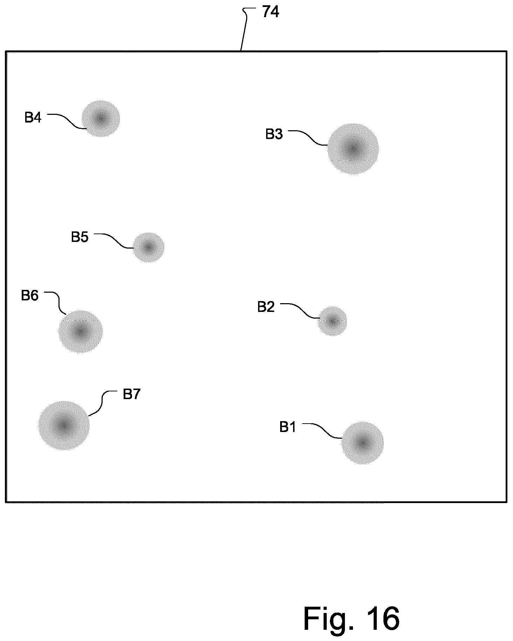

Fig. 16 illustrates one frame of image data captured by the camera 110 at time t=0. A series of spots B1-B7 of different sizes are seen across the extent of the LED layer 92. The amount of the charge deposited at each spot and thus the spot's intensity is different and is a function of the energy of the x-rays or particles that gave rise to the spots.

In any event, by tracking the location of each of the spots and resolving the size of the spot's bloom, and thus the energy of the photon or particle that gave rise to the bloom, a spatially resolved and spectrally (energy) resolved image of the object 214 is generated by the computer 224.

### Calibration

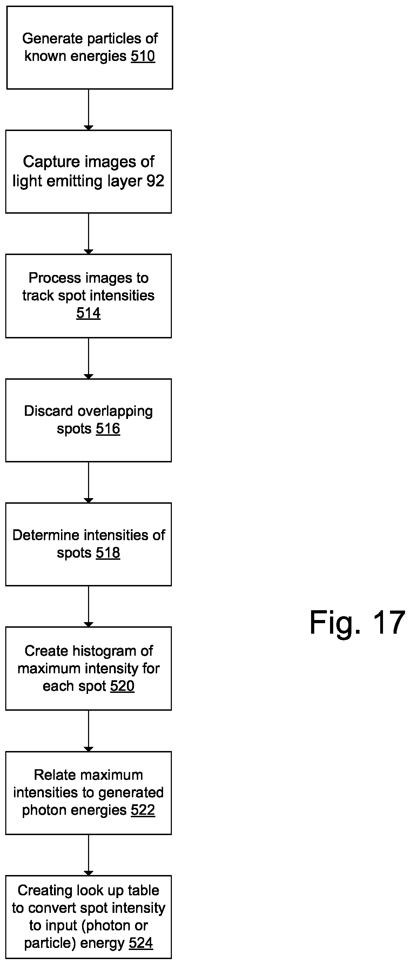

Fig. 17 is a flow diagram showing a method for calibrating the imaging spectrometer.

For the calibration, photons or particles of known energies are generated in step 510. A few energy values are needed for the calibration. **In** one example, the x-ray source system 202 is a set of radioisotopes with the right energy peaks in the detection energy ranges. In another example, the x-ray source system 202 is a tunable synchrotron monogenetic source.

The flux needs to be low so individual x-ray deposition events are sparsely distributed as shown in the Fig. 16. Many images of the light emitting layer 92 are captured by the camera 110 in step 512 and stored.

Next, the images are processed by the computer 224 to identify and characterize the individual spot positions and sizes within the conversion rate of the light emitting layer 92 in step 514. Any overlapping spots are discarded in step 516.

The relative intensities of the spots are determined in step 518 by the computer 224. This can be accomplished by taking the maximum detected spot intensity or total intensity of the spot area.

Then, in step 520, all the event intensity values of the spots are aggregated to form a histogram plot.

The peaks in the histogram are related to the corresponding input x-ray photon energy in step 522.

The foregoing steps can be repeated with different radioisotopes, for example, producing different input x-ray energies.

Then, an energy/intensity map 310 such as a lookup table (LUT) is produced to convert (with proper interpolation) the spot intensity to the input energy in step 524 by the computer 224.

The above calibration can be repeated for a few different bias and imaging conditions. The conditions include the bias amplitudes and transit rejection conditions.

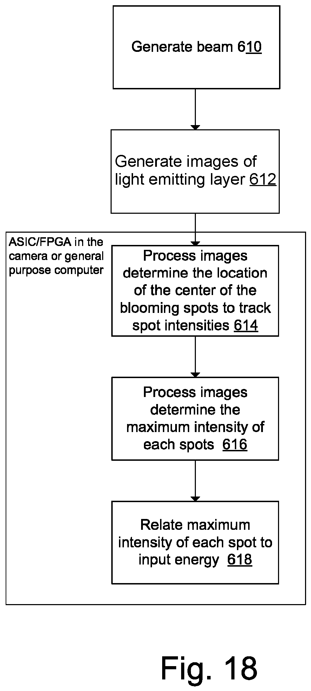

Fig. 18 describes how the calibrations are used to generate a spectrally and spatially resolved images or projections of the object 214.

Now, the system 200 is used to carry out the actual spectral imaging except energy of the x-ray (charged particle) source 202 is unknown.

Depending on the spectral accuracy requirements, the computer 224 resolve even with certain level of pileup conditions, i.e. the device can still work when the event spots are overlapping to a small degree. However, the mean spatial photon density/distribution should still be much smaller than the optical resolution of the system. Pileup rejection and correction algorithms can be applied to push the working flux level higher than calibration flux.

In any event, in step 610, the beam 205 is generated to illuminate the object 214.

In step 612, the camera 110 generates images of light emitting layer 92 of the detector 12. The images are processed to determine the location, center, of the blooming spots induced by the receipt of the photons or charged particles by the computer 224 in step 614.

In step 616, the images are processed by the computer 224 to determine the relative intensity of each of the spots using an identical method as in step 518.

Finally, the lookup table (LUT) is employed by the computer 224 to relate the spot intensity to the input energy in step 618.

Step 614 ~ 618 are performed in the computer system, but it need not be a unitary device. For example, the computer system could be a general purpose computer, in which the images have been downloaded and stored, or can be done in a dedicated ASIC/FPGA module as an add-on to the imaging camera or in a GPU. The advantages of such a module are speedup of processing and reduction in data volume and storage.

Of particular interest to the above is the use of deep learning based denoising for raw data processing by the computer 224 such as by denoising the images. Particularly noise-to-void / generally "blind spot" denoising techniques are useful, wherein the use of these techniques with employing self-supervised machine learning algorithms is preferred. They have a specific response to the spatial spectrum of noise that make them attractive. The features to be identified (signal spots) have an extended spatial extent whereas the noise (particularly camera read noise) has significant spatially white components. Blind spot denoising will be explicitly tailored to camera read noise removal and signal spot (non-white) recovery / retention. This can, in turn, be fed into subsequent algorithms (i.e. neural networks) for object (signal spot) identification and measurement.

According to a preferred embodiment, a deep learning based, blind spot denoising method is applied, wherein a true detection signal for any pixel is predicted based on surrounding pixels , whereas noise is assumed to be principally pixel wise (spatially) independent, i.e., to be uncorrelated between a pixel and its neighbors. In other words, it is assumed that the noise is spatially "white", i.e., is built up from equal contributions of all frequencies in the frequency domain. X-ray microscopy noise is usually constituted by combined X-ray shot (poisson) noise and a visible light shot (poisson) noise. As the X-ray shot process has to pass through a portion of the X-ray detector MTF (i.e. optics) this leads to a higher degree of spatial autocorrelation for the X-ray shot noise compared to other noise. In the present method, the spectral selectivity of blind spot denoising is thus advantageously applied to automatically remove the noise associated with one process (visible light (poisson) noise) while retaining spatially extended features of interest, such as a scintillation volume that is associated with a certain spot blooming event.

According to a preferred embodiment, preferably a U-net like structure is employed as a machine learning algorithm (neural network), particularly preferred a U-net like structure with standard regression loss (e.g. L2 norm). Further preferred, the input to the neural network is a single channel (i.e., the image data obtained by the camera), and the output of the neural network (after up- and down-convolution) is again a single channel (i.e., the denoised image data). Therein, in the denoised image data one or several pixels are removed from a region of pixels, as described in Krull et al, "Noise2Void - Learning Denoising from Single Noisy Images",arXiv:1811.10980*.* Further, the labels preferably are the set of pixels removed from the image for being noisy, and the loss is preferably computed on those pixels.

While this invention has been particularly shown and described with references to preferred embodiments thereof, it will be understood by those skilled in the art that various changes in form and details may be made therein without departing from the scope of the invention encompassed by the appended claims.

## Claims

1. A spectrally and spatially resolved x-ray and/or charged particle detection system (100), comprising:
a detector (12) comprising a semiconductor layer (74) configured for converting photons or particles into charge carriers and a light emitting layer (92) configured for generating light from the charge carriers;
an optical microscope (113, 116, 118) configured for reading out the detector (12);
a camera (110) coupled to the detector (12) by the optical microscope (113, 116, 118); and
a computer system that is configured to obtain images generated by the camera (110) and to track the response of the detector (12) to x-ray photons and/or charged particles.

2. The detection system (100) of claim 1, wherein the semiconductor layer (74) is configured to convert x-rays into the charge carriers.

3. The detection system (100) of either of claims 1 or 2, wherein the semiconductor layer (74) is amorphous selenium (a-Se), GaAs, CdZnTe, CdTe, or perovskite crystal (ABX3).

4. The detection system (100) of any of claims 1 to 3, wherein the light emitting layer (92) is an organic light emitting diode (OLED), GaAs, AlGaAs, InGaAs, CdTe or CdZnTe.

5. The detection system (100) of any of claims 1 to 4, wherein the light emitting layer (92) is disposed on an optical-side of the semiconductor layer (74), a x-ray side electrode layer (72) is deposited on an x-ray side opposite to the optical-side of the semiconductor detector layer (74), the light emitting layer (92) is disposed between the semiconductor layer (74) and an optical-side electrode layer (94) on the optical-side, and the detector (12) further comprises layers or structures that are configured to improve light outcoupling towards the optical-side electrode layer.

6. The detection system (100) of claim 5, wherein the layers or structures include a reflective layer, a Bragg grating (95), a surface structure, and/or microlens between the semiconductor layer and the light emitting layer.

7. The detection system (100) as claimed in any of claims 1 to 6, wherein the computer system is configured to locate spots induced by the x-ray photons or charged particles to resolve locations on the detector (12) and energy of the x-ray photons or charged particles that gave rise to the spots.

8. The detection system (100) as claimed in any of claims 1 to 7, wherein the computer system is configured to determine the energy of the x-ray photons or charged particles by reference to an energy/intensity map that relates a spot intensity to an energy of the received x-ray photons or charged particle.

9. The detection system (100) as claimed in claim 8, wherein an interval between successive frames captured by the camera (110) is less than 1 millisecond.

10. The detection system (100) as claimed in claim 1, wherein the detector (12) includes a dielectric material layer configured to shape a wavefront of the light generated from the light emitting layer (92).

11. A particle detection method, comprising:
converting x-ray photons and/or charged particles into electron-hole pairs as charge carrier types in a semiconductor layer (74) of a detector (12) comprising the semiconductor layer (74) configured for converting photons or particles into charge carriers;
converting one of the charge carrier types into photons in a light emitting layer (92) of the detector (12) comprising the light emitting layer (92) being configured for generating light from the charge carriers;
reading out the photons from the detector (12) with a camera (110) coupled to the detector (12); and
processing images generated by the camera (110) and tracking the response of the detector (12) to x-ray photons or charged particles to determine the position and the energy of the x-ray photons or charged particles.

12. The method of claim 11, employing the detection system (100) claimed in any of claims 1 to 10.

13. An imaging system (200), comprising:
an object stage system (210) configured for holding an object (214); and
a detection system (100) according to claim 1,
wherein the detector (12) is configured for detecting x-ray photons or charged particles from the object (214), and
wherein the computer system (224) is further configured to image the object (214) and to determine an energy of the x-ray photons or charged particles.

14. The imaging system (200) of claim 13, wherein the detection system (100) is further configured as claimed in any of claims 2 to 10.

15. A method for calibrating an x-ray photon or charged particle detection system (100), comprising:
generating x-ray photons or charged particles of known energy;
converting particles into electron-hole pairs as charge carrier types in a semiconductor layer (74) of a detector (12) comprising the semiconductor layer (74) configured for converting photons or particles into charge carriers;
converting one of the charge carrier types into photons in a light emitting layer (92) of the detector (12) comprising the light emitting layer (92) being configured for generating light from the charge carriers;
reading out the photons from the detector (12) with a camera (110); and
processing images generated by the camera (110) and tracking spots generated by the x-ray photons or charged particles received by the detector (12) and determining a relationship between the spots and the energy of the x-ray photons or charged particles.

16. The method of claim 14, employing the detector (12) claimed in any of claims 1 to 10.

17. The method of either of claims 15 or 16, wherein the processing images generated by the camera (110) includes employing deep learning to denoise the images.
